# EUROPEAN PATENT SPECIFICATION

(11) **EP 2 281 596 B1**
(45) Date of publication and mention of the grant of the patent: **03.10.2012**
(21) Application number: 09167331.9
(22) Date of filing: 06.08.2009
(51) Int. Cl.: A61M 16/06

(54) **Respiratory nasal mask with improved headgear connections**
Nasale Atemmaske mit verbesserten Kopfstückverbindungen
Masque respiratoire nasal doté de connexions de casque améliorées

(43) Date of publication of application: 09.02.2011
(73) Proprietor: Air Liquide Medical Systems, 92182 Antony Cedex (FR)
(72) Inventor: Rivetti, Alberto, 25038, ROVATO (BS) (IT); Alberici, Luca, 25128, BRESCIA (IT); Sandoni, Giuseppe, 25124, BRESCIA (IT)
(74) Representative: Pittis, Olivier

(56) References cited:
- EP-A1- 1 371 385
- EP-A2- 1 057 494
- WO-A2-03/082406
- US-A1- 2004 025 883
- US-A1- 2006 237 018
- US-A1- 2007 251 522
- US-B2- 7 017 576

## Description

The invention concerns a respiratory mask, in particular a nasal mask, with improved connection means for connecting the mask to a headgear, for use in the treatment of respiratory conditions or diseases, such as obstructive sleep apnea.

Nasal masks are commonly used for delivering non-invasive positive pressure ventilation (NPPV) or for nasal continuous positive airway pressure (N-CPAP) therapy of sleep disordered breathing (SDB) conditions, such as obstructive sleep apnea (OSA).

Nasal masks deliver a flow of breathable gas for or to assist patient respiration, especially during the night, i.e., when the patient is sleeping.

Such a mask assembly typically comprises a rigid or semi-rigid hollow shell defining a breathing chamber that receives at least a part of the patient's nose and further comprising a soft face-contacting cushion that comes into contact with the patient face, and a fore-head support and a headgear for correctly positioning, maintaining and/or securing the mask on the head of a patient. An example of a nasal mask of this kind is disclosed by EP-A-462701.

The shell typically receives a gas supply line which delivers a respiratory gas, such as air under pressure, into the breathing chamber of the shell. Further, the soft face-contacting cushion can be made of a soft, resilient elastomeric material that may conform to various facial contours, i.e., the patient face.

The mask assembly is secured to the wearer's head by straps or similar forming a headgear that can be adjusted to pull the mask against the face with sufficient force to achieve a gas tight seal between the mask and the wearer's face. The headgear is usually connected to the mask by means of connection means, such as slots arranged in the shell walls as taught by EP-A-462701, EP-A-874667 or WO-A-00/57942, or by means of more complicated release mechanism as disclosed by EP-A-1972357.

Other breathing mask is known from document WO 03/082406. Said mask comprises a shell defining an internal chamber and integrally formed connection means in form of two hooles for fixing a headgear.

Indeed, it is important for the patient's comfort and for getting an efficient treatment that gas leaks between the face cushion and the patient's face are avoided or at least very limited.

Thus, if any leak appears, then it can result in the pressure supplied to the entrance of the patient's airway being below the therapeutic value and the treatment becoming ineffective or insufficient.

Despite the fact that various mask configurations have already been proposed, obtaining a good positioning of the mask of the patient's face and head is still a problem in many cases. Indeed, many known headgear and mask configurations do not allow an easy fitting of the mask on the patient's face.

Further, many existing masks are also difficult to position on or to remove from the patient's face or head, in particular many existing headgear connection mechanisms are too complicated to use and often too expensive.

The main problem to be solved is to provide an improved nasal mask that is easy to put in place in a correct position and afterwards to remove, when the night is over, and which leads to a good sealing and thus to an efficient treatment, when it is in contact with the patient's face and used for treatment sleep disorders, such as OSA or similar.

The solution of the present invention is a respiratory mask according to claim 1.

The mask according to the present invention can further comprise one or more of the following additional features:
- the shell, the headgear-fixing hooks and the linking portions are made of a unique molded structure, made of polymer.
- the shell comprises an external border comprising the linking portions, preferably the external border has a triangular general shape.
- the shell further comprises a gas inlet for introducing a gas into the internal chamber.
- the shell comprises a gas inlet, and the two headgear-fixing hooks and the two linking portions are arranged symmetrically on opposite sides of the shell with respect to the plan of symmetry of the shell.
- the angle α formed between the axis AA, BB of each flat surface and the symmetry plan P of the shell is of between 5 and 40°, preferably of between 30° or less.
- the first axis AA of the first flat surface of the first headgear-fixing hook and the second axis BB of the second flat surface of the second headgear-fixing hook are coplanar.
- each headgear-fixing hook comprises a free end comprising a bent portion, preferably each bent portion projects toward the shell.
- each headgear-fixing hook comprises a free end comprising a bent portion projecting toward the shell.
- each headgear-fixing hook comprise locking/unlocking means for connecting to or disconnecting the headgear-fixing hook from the shell.
- the shell comprises an internal chamber delimited by a peripheral border and a cushion fixed to said peripheral border, said cushion having a central aperture for receiving at least part of the patient's nose, preferably said cushion comprises one or several flexible membranes.
- the shell further comprises a front support with additional headgear connection means, preferably said additional headgear connection means comprise one or several slots.

The invention also concerns an assembly formed by a headgear comprising straps and a mask according to the present invention, at least a first strap being fixed to at least one of the headgear-fixing hooks and at least a second strap being connected to one of the additional connection means

A preferred embodiment of a nasal mask according to the present invention is shown on the enclosed Figures, among which :
- Figures 1 and 2 represent front views of a mask according to the present invention,
- Figure 3 and 6 are enlarged views of the headgear-fixing hooks of the mask of Figures 1 and 2,
- Figures 4a and 4b represent the way of fixing the headgear to the headgear-fixing hooks of the mask of Figures 1 and 2, and
- Figure 5 shows the mask of Figures 1 and 2 in position on the patient's nose, and
- Figures 7 and 8 shows another embodiment of the headgear-fixing hooks of a mask according to the present invention.

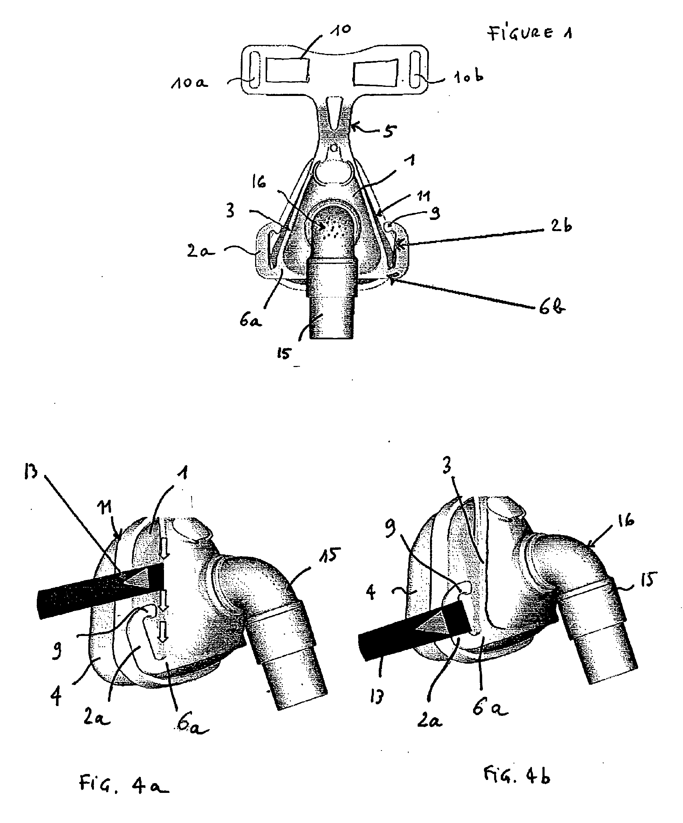

As illustrated in Figure 1, the respiratory mask according to the present invention comprises a rigid or semi-rigid hollow shell 1 defining an internal breathing chamber, wherein respiratory gas, such as air under pressure, is introduced via the inlet port 7 to which is connected a gas feeding line by means of a tubular connector 15.

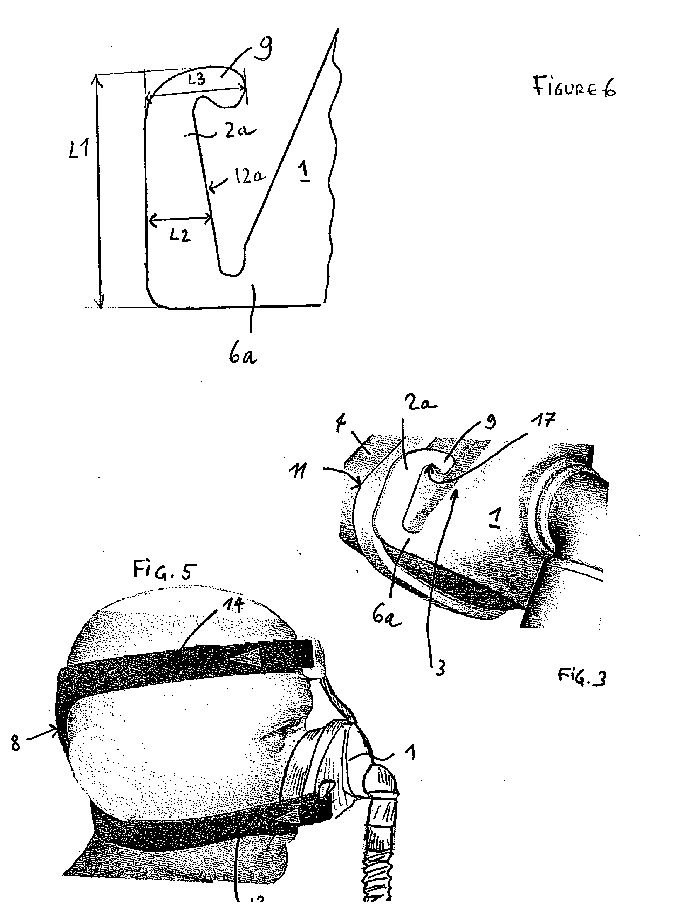

The inlet port 7 is arranged at the center of the shell 1 and through its wall thereby allowing air during pressure to be introduced in the breathing chamber. The shell 1 is preferably made of a polymer material, such as polycarbonate (PC), polypropylene (PP), ABS, nylon or polystyrene (PS), and is configured so as to be able to receive at least a part of the patient's nose. In other words, the patient introduces its nose into the internal volume of the breathing chamber of the shell 1 and breathes the pressurized gas contained therein. The mask further comprises a fore-head support 10 connected to the shell 1 by means of a strip portion 5, and a headgear 8 with straps 13, 14 for maintaining the mask in position on the head of the patient as represented in Figure 5. Such a mask structure is well known in the art and disclosed in many documents such as for instance EP-A-1334742, EP-A-462701, EP-A-1985327 or EP-A-956069.

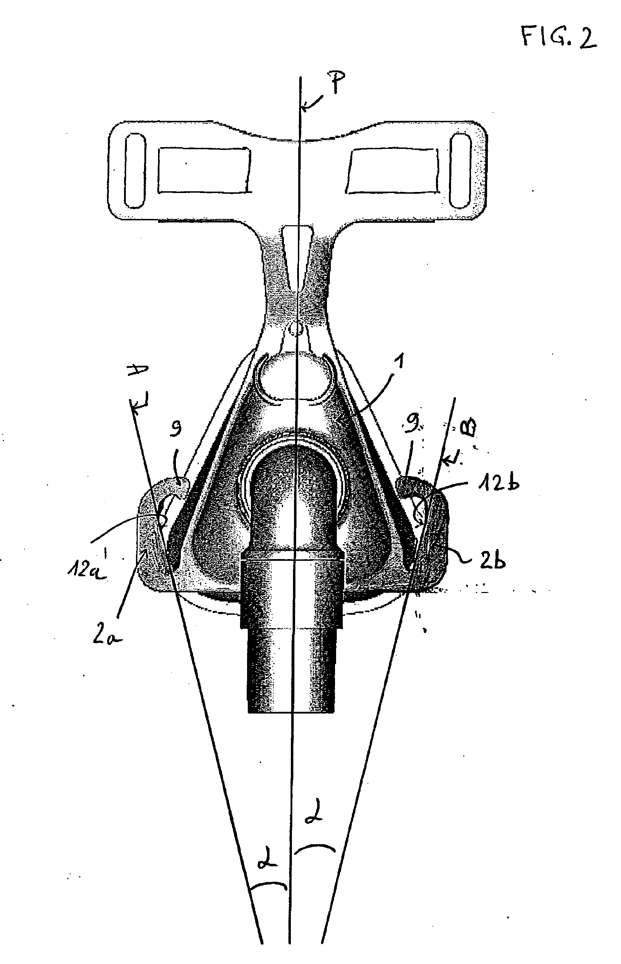

Further, as illustrated on Figure 2, in order to ensure a tight positioning of the nasal mask on the patient's face and to increase the comfort for the patient, the peripheral border or edge 11 of the shell 1 comprises a cushion 4 made of soft, resilient, elastomeric material that comes into contact with the patient face, said cushion 4 having a central aperture for receiving at least part of the patient's nose. More precisely, the border 11 and the cushion 4 have a general triangular or saddle-shape structure so as to match the contours of the nasal region of the patients and hence the cushion 4 comprises, roughly speaking, an upper nasal bridge region, a lower region and cheek regions connecting the nasal bridge and lower regions. In order to improve the seal and tightness, the cushion 4 can comprise one or several membranes, preferably two membranes. Such cushion 4 and shell 1 structures are well-known in the art and taught by many documents, such as EP-A-1334742, EP-A-264772, EP-A-956069, EP-A-874667, US-A-2,931,356 or EP-A-1479406.

As visible in Figures 1, 4a and 4b, the shell 1 is fluidly linked to a gas supply line (not shown) by means of a tubular connector 15, which delivers a respiratory gas, such as air under pressure 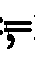 into the breathing chamber of the shell 1. The tubular connector 15 comprises one or several venting outlets, such a several holes 16, for venting to the atmosphere the CO₂-containing gases expired by the patient. The gas supply line is fed with air under pressure by a respiratory device (not shown).

Furthermore, several connection means 2a, 2b, 6a, 6b integral with the shell 1 are provided for fixing the headgear 8 to the shell 1. For instance, as shown in Figures 1 and 5, the frontal support 10 comprises two slots 10a, 10b for fixing the straps 14 of the headgear 8.

According to the present invention, in order to be able to easily put the mask in place in a correct position on the patient's nasal region, with a good air tightness and thus allowing obtaining an efficient treatment of sleep disorders, such as OSA or similar, some particular connection means 2a, 2b, 6a, 6b are provided on the shell 1.

More precisely, said connection means 2a, 2b, 6a, 6b comprise two headgear-fixing hooks 2a, 2b that are arranged on opposite sides of the shell 1, i.e., symmetrically on opposite sides of the shell 1 with respect to the gas inlet 7 and connector 15 as illustrated in Figures 1 and 2.

As detailed in Figures 1, 2 and 4a, each headgear-fixing hook 2a, 2b is fixed and made integral with the shell 1 body by means of a linking portion 6a, 6b, respectively.

As one can see on Figure 2, the headgear-fixing hooks 2a, 2b comprise flat portions, whereas the small linking portions 6a, 6b are also flat.

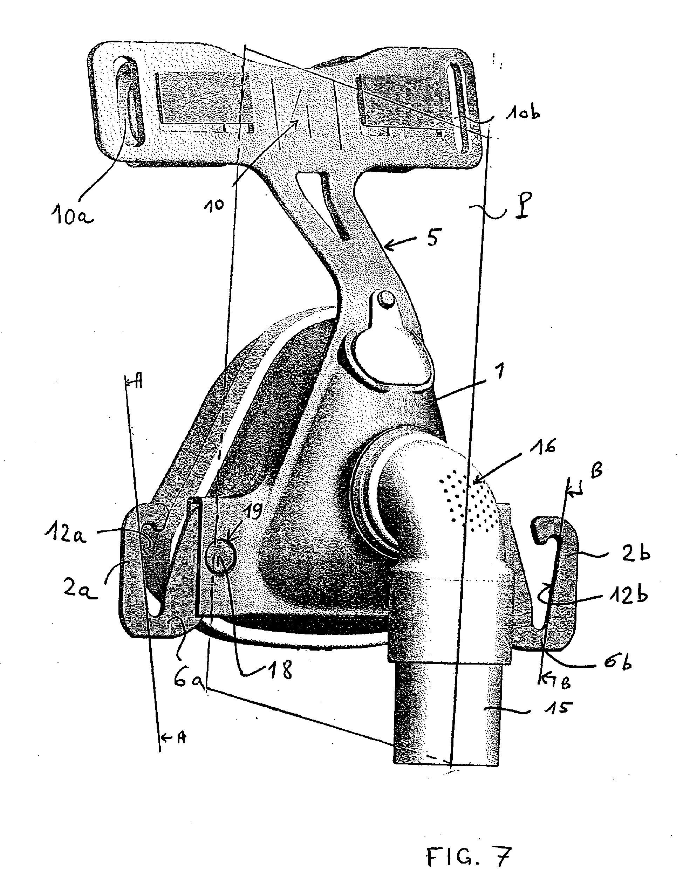

Actually, according to the present invention, each headgear-fixing hook 2a, 2b comprises a flat surface 12a, 12b facing the shell 1 of the mask. The flat surfaces 12a, 12b have each a corresponding axis AA, BB, respectively. The axis AA, BB of each flat surface 12a, 12b, respectively, form with the plan of symmetry P of the shell 1 an angle α of between 5 and 50°, preferably of less than 40°, as represented in Figures 2 and 7.

Indeed, such an angle α and structure allow having an efficient tightness and solve the above mentioned problems when the mask is positioned on the patient's face and the straps of the headgear 8 are fixed to the headgear-fixing hooks 2a, 2b as illustrated in Figures 4a and 4b.

Of course, the headgear-fixing hooks 2a, 2b and linking portions 6a, 6b can have other forms as long as the requirements regarding the angle between them are fulfilled.

Advantageously, the headgear-fixing hooks 2a, 2b, the linking portions 6a, 6b and the shell 1 are made of a unique molded structure, preferably a structure made of polymer that is injection-molded or thermo-molded for example.

In a preferred embodiment, the shell 1 comprises an external peripheral border 3 arranged around all or only a part of the periphery of shell 1, as shown in Figure 1 and detailed in Figure 3. Said external peripheral border 3 comprises the linking portions 6a, 6b, preferably the external peripheral border 3 has a general triangular shape. In other words, the linking portions 6a, 6b are part of the external peripheral border 3.

As shown in Figures 3 and 4a, each headgear-fixing hook 2a, 2b comprises a free end 9 comprising a bent portion 17 for retaining the strap 13 of the headgear 8, preferably each bent portion 17 projects toward the shell 1. In other words, each headgear-fixing hook 2a, 2b forms with its corresponding linking portion 6a, 6b a hook structure for the strap 13 of the headgear having a general "C" or of "[" shape as illustrated in Figure 3.

As shown in Figure 6, each headgear-fixing hooks 2a, 2b can have, for instance, a length L1 of between 1 and 5 cm, a lower width L2 of between 0.2 and 2 cm and a greater width L3 of between 0.5 and 4 cm, and a thickness of less than 80 mm.

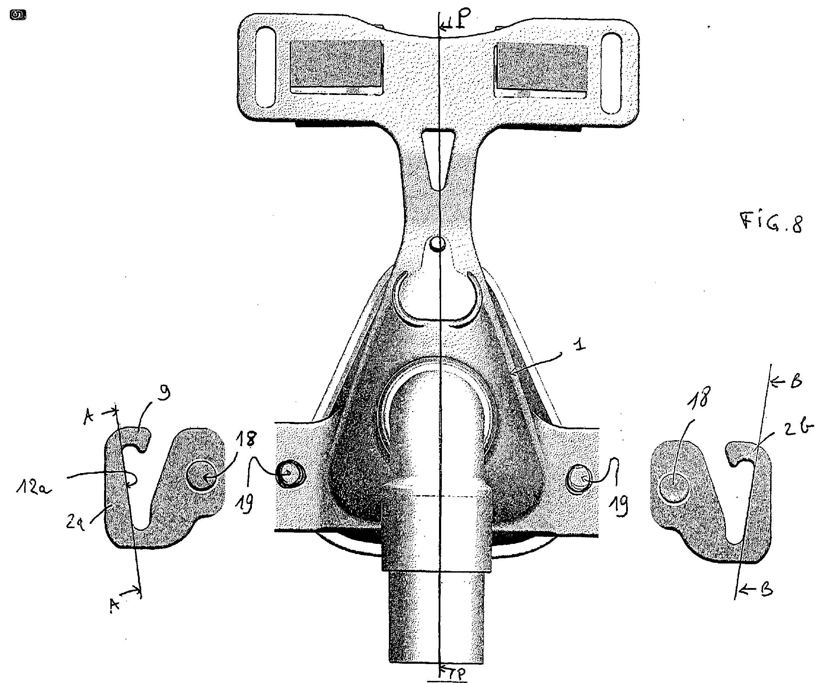

Further, Figures 7 and 8 shows another embodiment of the headgear-fixing hooks 2a, 2b of a mask according to the present invention, wherein said fixing hooks 2a, 2b are detachable, i.e. can be connect to the shell 1 or disconnected, by means of locking/unlocking means 18, 19. More precisely, the headgear-fixing hooks 2a, 2b can be detachable parts that can be inserted into corresponding lodgings arranged in the shell body 1 having adequate forms and structures, such as rectangular, round or others. The blocking of the headgear-fixing hooks 2a, 2b in their corresponding lodgings can be obtained thanks to a blocking system having for instance the form of a tooth 18 or similar that can be arranged either on parts 2a, 2b or on shell body 1, and that can cooperate with another structure, such as a receiving hole 19 or similar.

The insertion of parts 2a, 2b in their corresponding lodgings arranged in body 1 can be permanent (i.e. not detachable) or temporary (i.e. detachable).

The nasal mask of the present invention can be used in a method for treatment of a respiratory disorder or condition, for instance in non-invasive positive pressure ventilation (NPPV) or in a nasal continuous positive airway pressure (N-CPAP) therapy of sleep disordered breathing (SDB) conditions, such as obstructive sleep apnea (OSA).

## Claims

1. Respiratory mask comprising a shell (1) defining an internal chamber and connection means (2a, 2b, 6a, 6b) for fixing a headgear (8, 13, 14), said connection means (2a, 2b, 6a, 6b) being integral with the shell (1) and said shell (1) comprising a plan (P) of symmetry, said connection means (2a, 2b, 6a, 6b) comprising two headgear-fixing hooks (2a, 2b) arranged on opposite sides of the shell (1) and each being fixed to the shell (1) by means of a corresponding linking portion (6a, 6b), shell (1), the headgear-fixing hooks (2a, 2b) and the linking portions (6a, 6b) being made of a unique molded structure, **characterised in that** each headgear-fixing hook (2a, 2b) comprises a flat surface (12a, 12b) facing the shell (1), each flat surface (12a, 12b) having a corresponding axis (AA, BB) parallel to the flat surface, wherein the axis (AA, BB) of each flat surface (12a, 12b) forms with the symmetry plan (P) of the shell (1) an angle (α) of between 5 and 50°.

2. Mask according to claim 1, **characterized in that** the shell (1), the headgear-fixing hooks (2a, 2b) and the linking portions (6a, 6b) are made of a unique molded structure made of polymer.

3. Mask according to any one of the previous claims, **characterized in that** the shell (1) comprises an external border (3) comprising the linking portions (6a, 6b), preferably the external border (3) has a triangular general shape.

4. Mask according to any one of the previous claims, **characterized in that** the shell (1) further comprises a gas inlet (7) for introducing a gas into the internal chamber.

5. Mask according to any one of the previous claims, **characterized in that** the shell (1) comprises a gas inlet (7), and the two headgear-fixing hooks (2a, 2b) and the two linking portions (6a, 6b) are arranged symmetrically on opposite sides of the shell (1) with respect to the plan of symmetry of the shell (1).

6. Mask according to any one of the previous claims, **characterized in that** the angle (α) formed between the axis (AA, BB) of each flat surface (12a, 12b) and the symmetry plan (P) of the shell (1) is of between 5 and 40°, preferably of between 30° or less.

7. Mask according to any one of the previous claims, **characterized in that** the first axis (AA) of the first flat surface (12a) of the first headgear-fixing hook (2a) and the second axis (BB) of the second flat surface (12b) of the second headgear-fixing hook (2b) are coplanar.

8. Mask according to any one of the previous claims, **characterized in that** each headgear-fixing hook (2a, 2b) comprises a free end (9) comprising a bent portion (17), preferably each bent portion (17) projects toward the shell (1).

9. Mask according to any one of the previous claims, **characterized in that** each headgear-fixing hook (2a, 2b) comprises a free end (9) comprising a bent portion (17) projecting toward the shell (1).

10. Mask according to any one of the previous claims, **characterized in that** each headgear-fixing hook (2a, 2b) comprise locking/unlocking means (18, 19) for connecting to or disconnecting the headgear-fixing hook (2a, 2b) from the shell (1).

11. Mask according to any one of the previous claims, **characterized in that** the shell (1) comprises an internal chamber delimited by a peripheral border (11) and a cushion (4) fixed to said peripheral border (11), said cushion (4) having a central aperture for receiving at least part of the patient's nose, preferably said cushion (4) comprises one or several flexible membranes.

12. Mask according to any one of the previous claims, **characterized in that** the shell (1) further comprises a front support (10) with additional headgear connection means (10a, 10b), preferably said additional headgear connection means (10a, 10b) comprise one or several slots.

13. Assembly formed by a headgear (8, 13, 14) comprising straps (13, 14) and a mask according to any one of the previous claims, at least a first strap (13) being fixed to at least one of the headgear-fixing hooks (2a, 2b) and at least a second strap (14) being connected to one of the additional connection means (10a, 10b).

## Patentansprüche

1. Atemmaske, eine Hülle (1), die eine innere Kammer definiert, und Anschlussmittel (2a, 2b, 6a, 6b) zum Befestigen eines Kopfgeschirrs (8, 13, 14) umfassend, wobei die Anschlussmittel (2a, 2b, 6a, 6b) einstückig mit der Hülle (1) gebildet sind und die Hülle (1) eine Symmetrieebene (P) umfasst, wobei die Anschlussmittel (2a, 2b, 6a, 6b) zwei Kopfgeschirr-Befestigungshaken (2a, 2b) umfassen, die an gegenüberliegenden Seiten der Hülle (1) angeordnet und jeweils mittels eines entsprechenden Verbindungsabschnittes (6a, 6b) an der Hülle (1) befestigt sind, wobei die Hülle (1), die Kopfgeschirr-Befestigungshaken (2a, 2b) und die Verbindungsabschnitte (6a, 6b) aus einer im Ganzen ausgeformten Struktur hergestellt sind, **dadurch gekennzeichnet, dass** jeder Kopfgeschirr-Befestigungshaken (2a, 2b) eine ebene Fläche (12a, 12b) umfasst, die der Hülle (1) zugewandt ist, wobei jede ebene Fläche (12a, 12b) eine entsprechende, parallel zur ebenen Fläche verlaufende Achse (AA, BB) aufweist, wobei die Achse (AA, BB) jeder ebenen Fläche (12a, 12b) mit der Symmetrieebene (P) der Hülle (1) einen Winkel (α) zwischen 5 und 50° bildet.

2. Maske nach Anspruch 1, **dadurch gekennzeichnet, dass** die Hülle (1) die Kopfgeschirr-Befestigungshaken (2a, 2b) und die Verbindungsabschnitte (6a, 6b) aus einer im Ganzen ausgeformten Struktur aus Polymer hergestellt sind.

3. Maske nach einem der vorhergehenden Ansprüche, **dadurch gekennzeichnet, dass** die Hülle (1) eine Außenbegrenzung (3) umfasst, welche die Verbindungsabschnitte (6a, 6b) umfasst, wobei die Außenbegrenzung (3) vorzugsweise eine im Allgemeinen dreieckige Form aufweist.

4. Maske nach einem der vorhergehenden Ansprüche, **dadurch gekennzeichnet, dass** die Hülle (1) ferner einen Gaseinlass (7) zum Einführen eines Gases in die innere Kammer umfasst.

5. Maske nach einem der vorhergehenden Ansprüche, **dadurch gekennzeichnet, dass** die Hülle (1) einen Gaseinlass (7) umfasst und die zwei Kopfgeschirr-Befestigungshaken (2a, 2b) und die zwei Verbindungsabschnitte (6a, 6b) im Verhältnis zur Symmetrieebene der Hülle (1) symmetrisch auf gegenüberliegenden Seiten der Hülle (1) angeordnet sind.

6. Maske nach einem der vorhergehenden Ansprüche, **dadurch gekennzeichnet, dass** der Winkel (α), der zwischen den Achsen (AA, BB) jeder ebenen Fläche (12a, 12b) und der Symmetrieebene (P) der Hülle (1) gebildet ist, zwischen 5 und 40° beträgt, vorzugsweise zwischen 30° oder weniger.

7. Maske nach einem der vorhergehenden Ansprüche, **dadurch gekennzeichnet, dass** die erste Achse (AA) der ersten ebenen Fläche (12a) des ersten Kopfgeschirr-Befestigungshakens (2a) und die zweite Achse (BB) der zweiten ebenen Fläche (12b) des zweiten Kopfgeschirr-Befestigungshakens (2b) auf gleicher Ebene liegen.

8. Maske nach einem der vorhergehenden Ansprüche, **dadurch gekennzeichnet, dass** jeder Kopfgeschirr-Befestigungshaken (2a, 2b) ein freies Ende (9) umfasst, das einen gekrümmten Abschnitt (17) umfasst, wobei jeder gekrümmte Abschnitt (17) vorzugsweise hin zur Hülle (1) ragt.

9. Maske nach einem der vorhergehenden Ansprüche, **dadurch gekennzeichnet, dass** jeder Kopfgeschirr-Befestigungshaken (2a, 2b) ein freies Ende (9) umfasst, das einen gekrümmten Abschnitt (17) umfasst, der hin zur Hülle (1) ragt.

10. Maske nach einem der vorhergehenden Ansprüche, **dadurch gekennzeichnet, dass** jeder Kopfgeschirr-Befestigungshaken (2a, 2b) Ver-/Entriegelungsmittel (18, 19) zum Anschließen oder zum Lösen der Kopfgeschirr-Befestigungshaken (2a, 2b) an die beziehungsweise von der Hülle (1) umfasst.

11. Maske nach einem der vorhergehenden Ansprüche, **dadurch gekennzeichnet, dass** die Hülle (1) eine innere Kammer, die durch eine periphere Begrenzung (11) begrenzt ist, und ein Polster (4) umfasst, das an der peripheren Begrenzung (11) befestigt ist, wobei das Polster (4) eine Mittelöffnung aufweist, um zumindest einen Teil der Nase eines Patienten aufzunehmen, wobei das Polster (4) vorzugsweise eine oder mehrere flexible Membranen umfasst.

12. Maske nach einem der vorhergehenden Ansprüche, **dadurch gekennzeichnet, dass** die Hülle (1) ferner eine vordere Abstützung (10) mit zusätzlichen Kopfgeschirr-Befestigungsmitteln (10a, 10b) umfasst, wobei die zusätzlichen Kopfgeschirr-Befestigungsmittel (10a, 10b) vorzugsweise einen oder mehrere Schlitze umfassen.

13. Anordnung, gebildet aus einem Kopfgeschirr (8, 13, 14), die Gurte (13, 14) und eine Maske nach einem der vorhergehenden Ansprüche umfasst, wobei mindestens ein erster Gurt (13) an mindestens einem der Kopfgeschirr-Befestigungshaken (2a, 2b) befestigt ist und mindestens ein zweiter Gurt (14) an einem der zusätzlichen Befestigungsmittel (10a, 10b) angeschlossen ist.

## Revendications

1. Masque respiratoire comprenant une gaine (1) définissant une chambre interne et des moyens de raccordement (2a, 2b, 6a, 6b) pour fixer un harnais de tête (8, 13, 14), lesdits moyens de raccordement (2a, 2b, 6a, 6b) étant formés d'une seule pièce avec la gaine (1) et ladite gaine (1) comprenant un plan de symétrie (P), lesdits moyens de raccordement (2a, 2b, 6a, 6b) comprenant deux crochets (2a, 2b) de fixation du harnais de tête agencés sur les côtés opposés de la gaine (1) et chacun étant fixé à la gaine (1) au moyen d'une partie de liaison correspondante (6a, 6b), la gaine (1), les crochets (2a, 2b) de fixation du harnais de tête et les parties de liaison (6a, 6b) étant constitués d'une structure moulée unique, **caractérisé en ce que** chaque crochet (2a, 2b) de fixation du harnais de tête comprend une surface plate (12a, 12b) tournée vers la gaine (1), chaque surface plate (12a, 12b) ayant un axe correspondant (AA, BB) parallèle à la surface plate, dans lequel l'axe (AA, BB) de chaque surface plate (12a, 12b) forme avec le plan de symétrie (P) de la gaine (1) un angle (α) compris entre 5 et 50 °.

2. Masque selon la revendication 1, **caractérisé en ce que** la gaine (1), les crochets (2a, 2b) de fixation du harnais de tête et les parties de liaison (6a, 6b) sont constitués d'une structure moulée unique formée d'un polymère.

3. Masque selon l'une quelconque des revendications précédentes, **caractérisé en ce que** la gaine (1) comprend un bord externe (3) comprenant les parties de liaison (6a, 6b), le bord externe (3) ayant de préférence une forme générale triangulaire.

4. Masque selon l'une quelconque des revendications précédentes, **caractérisé en ce que** la gaine (1) comprend en outre une entrée de gaz (7) pour introduire un gaz dans la chambre interne.

5. Masque selon l'une quelconque des revendications précédentes, **caractérisé en ce que** la gaine (1) comprend une entrée de gaz (7) et deux crochets (2a, 2b) de fixation du harnais de tête et les deux parties de liaison (6a, 6b) sont aménagées symétriquement sur les côtés opposés de la gaine (1) par rapport au plan de symétrie de la gaine (1).

6. Masque selon l'une quelconque des revendications précédentes, **caractérisé en ce que** l'angle (α) formé entre l'axe (AA, BB) de chaque surface plate (12a, 12b) et le plan de symétrie (P) de la gaine (1) se situe entre 5 et 40 °, de préférence de 30 ° ou moins.

7. Masque selon l'une quelconque des revendications précédentes, **caractérisé en ce que** le premier axe (AA) de la première surface plate (12a) du premier crochet (2a) de fixation du harnais de tête et le second axe (BB) de la seconde surface plate (12b) du second crochet (2b) de fixation du harnais de tête sont coplanaires.

8. Masque selon l'une quelconque des revendications précédentes, **caractérisé en ce que** chaque crochet (2a, 2b) de fixation du harnais de tête comprend une extrémité libre (9) comprenant une partie cintrée (17), chaque partie cintrée (17) faisant de préférence saillie vers la gaine (1).

9. Masque selon l'une quelconque des revendications précédentes, **caractérisé en ce que** chaque crochet (2a, 2b) de fixation du harnais de tête comprend une extrémité libre (9) comprenant une partie cintrée (17) faisant saillie vers le gaine (1).

10. Masque selon l'une quelconque des revendications précédentes, **caractérisé en ce que** chaque crochet (2a, 2b) de fixation du harnais de tête comprend des moyens de verrouillage/déverrouillage (18, 19) pour raccorder le crochet (2a, 2b) de fixation du harnais de tête à la gaine (1) ou l'en dégager.

11. Masque selon l'une quelconque des revendications précédentes, **caractérisé en ce que** la gaine (1) comprend une chambre interne délimitée par un bord périphérique (11) et un amortisseur (4) fixé audit bord périphérique (11), ledit amortisseur (4) ayant une ouverture centrale pour recevoir au moins une partie du nez du patient, ledit amortisseur (4) comprenant de préférence une ou plusieurs membranes flexibles.

12. Masque selon l'une quelconque des revendications précédentes, **caractérisé en ce que** la gaine (1) comprend par ailleurs un support frontal (10) avec des moyens supplémentaires (10a, 10b) de raccordement du harnais de tête, lesdits moyens supplémentaires (10a, 10b) de raccordement du harnais de tête comprenant de préférence une ou plusieurs fentes.

13. Ensemble formé d'un harnais de tête (8, 13, 14) comprenant des sangles (13, 14) et un masque selon l'une quelconque des revendications précédentes, au moins une première sangle (13) étant fixée à au moins l'un des crochets (2a, 2b) du harnais de tête et au moins une seconde sangle (14) étant raccordée à l'un des moyens supplémentaires de raccordement (10a, 10b).
